(19) Europäisches Patentamt
European Patent Office
Office européen des brevets

(11) Publication number: **0 116 222**
**A1**

(12) # EUROPEAN PATENT APPLICATION

(21) Application number: 83307715.9

(22) Date of filing: 19.12.83

(51) Int. Cl.³: C 07 H 15/24
A 61 K 31/00

(30) Priority: 20.12.82 US 450863

(43) Date of publication of application:
22.08.84 Bulletin 84/34

(84) Designated Contracting States:
AT BE CH DE FR GB IT LI NL SE

(71) Applicant: THE OHIO STATE UNIVERSITY RESEARCH FOUNDATION
1314 Kennear Road
Columbus Ohio 43212(US)

(72) Inventor: Priebe, Waldemar
659 Stinchcomb Drive Apt. 1
Columbus Ohio(US)

(72) Inventor: Horton, Derek
1500 McCoy Road
Columbus Ohio 43220(US)

(72) Inventor: Wolgemuth, Richard L.
10202 Kimberly Drive
Plain City Ohio 43064(US)

(74) Representative: Warren, Keith Stanley et al,
BARON & WARREN 18 South End Kensington
London W8 5BU(GB)

(54) **14-Acyloxy-2'-halo-anthracycline anti-cancer antibiotics.**

(57) Compounds of the formula (I) and pharmaceutical preparations containing the same are disclosed

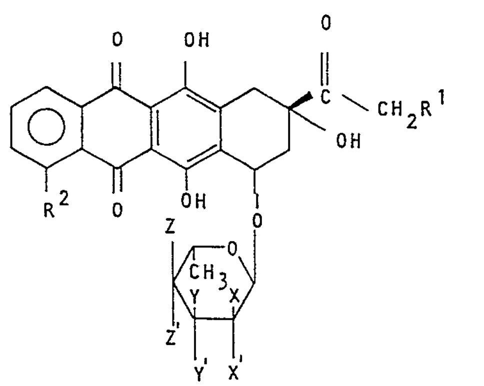

wherein $R^1$ is $-OOCR^3$ or $-OOC(CH_2)_nCOOR^4$; $R^2$ is hydrogen, hydroxy or methoxy; one of X and X' is a halogen atom selected from the group consisting of fluorine, chlorine, bromine and iodine and the other is hydrogen; one of Y and Y' is hydrogen and the other is selected from the group consisting of hydrogen, hydroxy and acyloxy; one of Z and Z' is hydrogen and the other is selected from the group consisting of hydrogen, hydroxy and acyloxy; $R^3$ is an alkyl group containing 1 to 8 carbon atoms; $R^4$ is a hydrogen atom, a metal atom, or an alkyl group containing 1 to 4 carbon atoms; and n is an integer of 0 to 6. These compounds are effective in treating a broad spectrum of neoplastic conditions including leukemias, lymphomas, and various tumors.

EP 0 116 222 A1

14-Acyloxy-2'-Halo-Anthracycline Anti-Cancer Antibiotics

Anthracyline antibiotics including doxorubicin, daunorubicin, and carminomycin have emerged as important chemotherapeutic agents in the treatment of a broad spectrum of neoplastic conditions including acute myeloblastic and lymphoblastic leukemias. Doxorubicin (also known as Adriamycin) is the subject of U.S. Patent No. 3,590,028 and is a prescribed antineoplastic agent used in a number of chemotherapeutic treatments.

Certain undesirable side effects have limited the usefulness of known anthracyline antibiotics. One of their more serious side effects, however, is their cardiotoxicity which severely restricts the dosages and the frequency with which the antibiotic can be administered and, in turn, limits their overall effectiveness as an antibiotic. Many of the other side effects which accompany the administration of these agents can be managed by administering other pharmaceutical agents in combination with them, however, the cardiopathic effects are not easily controlled or reversed.

In view of the proven effectiveness of known anthracyclines in the treatment of cancer, efforts have been undertaken to develop less toxic derivatives which can be administered in high, more effective dosages with greater frequency. The compounds disclosed in U.S. Patent No. 4,201,773 to Horton et al are among anthracyline derivatives that have been proposed to have better therapeutic ratios than their naturally occurring counterparts. These compounds are derivatives of Adriamycin, daunomycin, and demethoxydaunomycin in which the 3'amino group in the sugar moiety is substituted with a hydroxy group. Horton et al and Prieke also disclose a

number of 2'-halo derivatives of Adriamycin, daunomycin and demethoxydaunomycin in their U.S. Patent Application Serial No. 268,623 filed May 29, 1981 and refiled August 17, 1982 as U.S. Patent Application Serial No. 408,942.

One of the disadvantages of the aforementioned compounds is that they have relatively low solubility in water. This limits their usefulness and effectiveness because it often necessitates that they be administered in large volumes of infusate over a period of hours. Furthermore, in view of their low water solubility, the compounds are difficult to administer in amounts which would be effective in the treatment of some cancers.

Thus, there is a need for less toxic anthracyline antibiotics which are water soluble and readily absorbed into the blood stream.

---

The present invention relates to a novel anthracyline antibiotic which is represented by the formula (I)

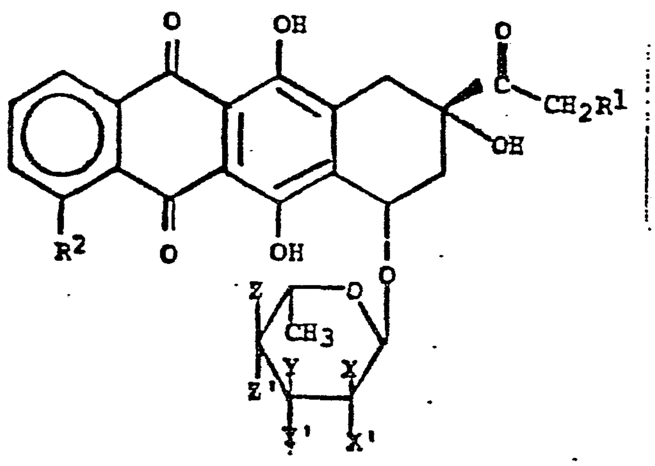

wherein $R^1$ is $-OOCR^3$ or $-OOC(CH_2)_nCOOR^4$; $R^2$ is hydrogen, hydroxy or methoxy; one of X and X' is a halogen atom selected from the group

consisting of fluorine, chlorine, bromine, and iodine and the other is hydrogen; one of Y and Y' is hydrogen and the other is selected from the group consisting of hydrogen, hydroxy and acyloxy; one of Z and Z' is hydrogen and the other is selected from the group consisting of hydrogen, hydroxy and acyloxy; $R^3$ is an alkyl group containing 1 to 8 carbon atoms; $R^4$ is a hydrogen or metal atom (for example an alkali metal atom such as Na, K, and the like), or an alkyl group containing 1 to 4 carbon atoms, and n is an integer of 0 to 6.

The present invention also provides pharmaceutical preparations containing the aforesaid compounds in suitable carriers and in therapeutically effective amounts.

In a more particular embodiment, the present invention provides compounds of the formula (I) wherein $R^2$ is hydrogen, hydroxy or methoxy; one of X and X' (preferably X') is bromine or iodine; one of Y and Y' (preferably Y') is acetoxy or hydroxy; one of Z and Z' is acetoxy or hydroxy and $R^1$ is represented by the formula $-OOCR^3$ or $-OOC(CH_2)_nCOOR^4$ wherein $R^4$ is a hydrogen or metal atom, the balance of the substitutes being defined as above.

In a still more particular embodiment, the present invention provides compounds of the formula (I) wherein $R^2$ is methoxy; X' is iodine; Y' is acetoxy or hydroxy, Z or Z' is acetoxy or hydroxy and $R^1$ is hemiglutaryloxy, hemiadipyloxy, or acetoxy.

The compounds of the present invention are 14-acyloxy-2'-halo-3'deamino derivatives of the antibiotics doxorubicin, carminomycin, and

4-demethoxydaunomycin.

The anthracycline derivatives of the present invention are glycosides made up of an anthracylcinone substituted with an acyloxy group at C-14 which is coupled at C-7 to a 2,6-dideoxy-2-halo-hexopyranose sugar. In general, these compounds are prepared by two alternative routes:

1) By reacting 1,5-anhydro-2,6-dideoxy-hex-1-enitols (6-deoxy-glycals) with an equimolar amount of a 14-O-acyl-aglycon in the presence of N-halogeno-succinimide to form the 2-halo glycoside, or by reacting the appropriate glycals with halogen and then coupling them with anthracyclinones by using known coupling reagents (for example silver trifluoromethanesulfonate).

2. By reacting the sugar with daunomycinone or 4-demethoxydaunomycinone under the above described conditions followed by functionalization of position 14 by halogenation (For example bromination in an inert solvent) and reacting the product with a sodium or potassium salt of a fatty acid of the formula $R^3COOH$ where $R^3$ is defined as above analogous to the teachings in U.S. Patent No. 3,803,124. Where $R^1$ is a dicarboxylic acid moiety, the monosodium salt of the dicarboxylic acid may be used as above. The 14-O-hemiglutarate and the 14-O-hemiadipate derivatives of the present invention can be prepared by analogy to the teachings in U.S. Patent No. 4,299,822.

The compounds of the present invention are preferably prepared from 1,6-anhydro-3,4-di-O-acetyl-2,6-dideoxy-hex-1-enitols such as 3,4-di-O-acetyl-L-fucal or 3,4-di-O-acetyl-L rhamnal. These sugars can be prepared as described

in B. Iselin and T. Reichstein, Helv. Chim. Acta, 27, 1146, 1200 (1944). The aglycon is usually reacted with 3,4-di-O-acetyl-L-rhamnal or 3,4,-di-O-acetyl-L-fucal in an anhydrous mixture of acetonitrile and tetrahydrofuran followed by the addition of a halogenating agent such as N-iodosuccinimide or N-bromosuccinimide. The halogenating agent is generally used in a stoichiometric excess, e.g., 1.5 to 3 times the amount of the aglycon on a molar basis.

The synthesis of the compounds of the present invention will now be illustrated in more detail by the following examples:

## Example 1

Preparation of 14-O-acetyl-7-O-(3,4-di-O-acetyl-2,6-dideoxy-2-iodo- α -L-*manno*-hexopyranosyl) adriamycinone.

0.92 mmol (198 mg) of 3,4-di-O-acetyl-L-rhamnal and 0.52 mmol (239 mg) of 14-O acetyladriamycinone (prepared in accordance with U.S. Patent No. 3,803,124) were added to a mixture of dry acetonitrile (14 ml) and tetrahydrofuran (7 ml). The reaction mixture was flushed with dry argon and cooled to 0°C while N-iodosuccinimide in an amount of 1.52 mmol (343 mg) was added thereto. The mixture was stirred at room temperature for 44 hours.

Thin layer chromatography (3:1 toluene-acetone) was performed on precoated plastic sheets (0.2 mm) coated with silica gel 60 F-254 (E. Merck, Darmstadt, G.F.R.) and showed the presence of one major and one less-polar minor product. Traces of substrate were still present. The mixture was diluted with dichloromethane (100 ml) and shaken twice with 10% aqueous sodium thiosulfate (50 ml),

and washed twice with an excess of water. The organic layer was dried with $MgSO_4$. Filtration and evaporation of the solvent gave a thick, dark, red syrup which was dissolved in chloroform and evaporated onto silica gel (3 g). The red powder thus obtained was placed on a column of silica gel 60 (230--400 mesh) (E. Merck, Darmstadt, G.F.R.). (30 g in 2:1 hexane-ethyl acetate). The column was eluted first with 2:1 hexane-ethyl acetate (200 ml) and then with 8:1 toluene-acetone. Two fractions were obtained. The more-polar one was isolated and fully characterized after crystalization from acetone, ethyl ether, and hexane, m.p. 130-135°C; $[\alpha]_D^{21}$ +94° (*c* 0.02, chloroform); $\nu_{max}^{KBr}$ 3380 (OH), 1745-1720 (C=0), 1610 and 1575 $cm^{-1}$ (chelated quinone); $^1$H-n.m.r. ($CDCl_3$, 200 MHZ): δ 13.95, 13.15 (s, 1H, HO-6, HO-11), 8.00 (dd, 1H, $\underline{J}_{1,2}$ 7.7, $\underline{J}_{1,3}$ 1.1 Hz, H-1), 7.77 (apparent, 1H, H-2), 7.39 (dd, 1H, $\underline{J}_{2,3}$ 8.5 Hz H-3), 5.78 (bs, 1H, H-1'), 5.34 (d, 1H, $\underline{J}_{14A,\ 14B}$ 18.4 Hz, H-14A), 5.26 (m, 1H, H-7), 5.18 (t, 1H, $\underline{J}_{3',4'}$ =$\underline{J}_{4',5'}$ 9.5 Hz, H-4'), 5.12 (d, 1H, H-14B), 4.59 (dd, 1H, $\underline{J}_{1',2'}$ 1.5, $\underline{J}_{2',3'}$ 4.4 Hz, H-2'), 4.33 (dd, 1H, H-3'), 4.16 (s, 1H, HO-9), 4.10 (dq, 1H, H-5'), 4.08 (s, 3H, OMe), 3.27 (dd, 1H, $\underline{J}_{8e,\ 10e}$ 1.5 Hz, H-10e), 2.93 (d, 1H, $\underline{J}_{10e,10ax}$ 19.1 Hz, H-10ax), 2.45 (bd, 1H, $\underline{J}_{8e,8ax}$ 15.1 Hz, H-8e), 2.21-2.07 (m, 1H, H-8ax), 2.21, 2.07, 2.04 (s, 3H, OAc), 1.31 (d, 3H $\underline{J}_{5',6'}$ 6.25, H-6'); $^{13}$C-n.m.r. ($CDCl_3$, 50 Mhz): δ 206.6 (C-13), 187.4, 187.1 (C-5, C-12), 170.6, 169.9 (C=O), 161.4 (C-4), 156.3, 155.8 (C-6, C-11), 135.9 (C-2), 135.7, 134.0, 132.9 (C-6a, C-10a, C-12a), 121.1 (C-4a), 120.0 (C-1), 118.7 (C-3), 111.9, 111.8 (C-5a, C-11a), 104.7 (C-1'), 76.6 (C-9, signal strongly

overlap with $CDCl_3$ signals), 72.4 (C-4'), 70.6 (C-7), 69.1 (C-3'), 68.5 (C-5'), 65.9 (C-14), 56.7 (OMe), 35.5 (C-8), 33.6 (C-10), 29.0 (C-2'), 20.7, 20.6, 20.3 (OAc), 17.3 (C-6').

<u>Anal</u>. Calc. for $C_{33}H_{33}IO_{15}$ (796.526): C, 49.76; H, 4.18; I, 15.93. Found: C, 49.65; H, 4.36.

## <u>Example 2</u>

Preparation of 7-O-(3,4-di-O-acetyl-2,6-dideoxy-2-iodo-α-L-manno-hexopyranosyl)-14-O-(5-carboxypentanoyl) adriamycinone.

1.385 mmol (1.023g) of 7-O-(3,4-di-O-acetyl-2,6-dideoxy-2-iodo-α-L-manno-hexopyranosyl) daunamycinone was dissolved in chloroform. Then 1.75 g of bromine in 10 ml chloroform was added. The reaction was monitored by TLC (toluene:acetone 4:1), and after 6 hours no substrate was present. The sample was concentrated on the rotary evaporator and evaported twice with chloroform. Then the sample was crystallized from chloroform-ethyl ether-hexane to provide 842 mg of 14-bromo-7-O-(3,4-di-O-acetyl-2,6-dideoxy-2-iodo-α-L-manno-hexopyranosyl) daunomycinone.

289.2 mg. (0.354 mmol) of the 14-bromo compound was dissolved in 2,4-pentanedione (60 ml), then 1.2 g of monosodium adipate was added and was refluxed for 40 min. After the reaction mixture reached room temperature methylene chloride (300 ml) was added and the solution was filtered, washed several times with water and dried over magnesium sulfate. Chromatography afforded 130 mgs. (41.6%) of red foam which was crystallized from chloroform-ethyl ether-hexane. Yield: 74 mg (23.7%), m.p. 125-130°, $[\alpha]^{26}$ + 94° (<u>c</u> 0.02, chloroform); $\nu_{max}^{KBr}$ 3470 (OH), 1735 (bs), 1618, and 1577 $cm^{-1}$

BAD ORIGINAL

(chelated quinone); 'H-n.m.r. ($CoCl_3$, 300 MHz): δ 13.92, 13.10 (S, 1H, HO-6, 11), 7.97 (d, 1H, $\underline{J}_{1,2}$ 7.4 Hz, H-1), 7.76 (app. t, 1H, H-2) 7.37 (d, 1H, $\underline{J}_{2,3}$ 8.1 Hz, H-3), 5.78 (s, 1H, H-1'), 5.32 (d, 1H, $\underline{J}_{14a,14b}$ 18.2 Hz, H-14A), 5.28 (m, 1H, H-7), 5.18 (t, 1H, $\underline{J}_{3',4'}$=$\underline{J}_{4',5'}$ 9.4- Hz, H-4'), 5.15 (d, 1H, H-14B), 4.59 (d, 1H, $\underline{J}_{2',3'}$ + 4.3Hz, H-2'), 4.3 + (dd, 1H, H-3'), 4.08 (m, H-5, $OCH_3$), 3.31 (dd, 1H, $\underline{J}_{8e,10e}$ 1.1 Hz, H-10e), 2.99 (d, 1H, $\underline{J}_{10e,10ax}$), 19.0 Hz, H-10ax), 2.4-6 (m, 5H, $CH_2$, H-8e), 2.19-2.06 (m, 1H, H-8ax), 2.07, 204 (S. 3H, Ohe), 1.76 (M, 4-H, $CH_2$) and 1.31 (d, 3H, $\underline{J}_{5',6'}$ 6.3 Hz, H-6'), 13C-n.m.r. ($CDCl_3$, 50 MHz); δ 206.5 (C-13), 187.1, 186.9 (C-5, C-12), 175.1, 172.9, 169.9, 169.8 (==0), 161.3 (C-4), 156.2, 155.7 (C-6, C-11), 135.9 (C-2), 135.6, 134-0, 132.9 (C-6r, 10r, 12r), 120.9 (C-4r), 119.9 (C-1), 118.7 (C-3), 111.8, 111.7 (C-5a, 11a), 104.6 (C-1'), 77.0 (C-9, overlap with $CDCl_3$), 72.5 (C-4'), 70.6 (C-7), 69.1 (C-3'), 68.5 (C'-5'), 65.8 (C-14), 56.6 (OMe), 35.4' (C-8), 33.5, 33.4, 33.3 (C-10, $CH_2$) 29.1 (C-2'), 24.1, 23.9 ($CH_2$), 20.7, 20.6 (OAc), 17.3 (C-6').

Therepeutic compositions containing the novel compounds of the present invention as active agents can be prepared by dispersing or dissolving the compound in any pharmecutically acceptable non-toxic carrier suitable for the desired mode of administration. Therepeutic compositions of the present invention may be administered parenterally by intravenous, intramuscular, intraperitoneal, or other conventional injection or orally in some cases. Preferably, the carrier is an aqueous medium buffered to pH 7.2-7.5, the physiological range. Any suitable conventional buffer can be used such as tris phosphates, bicarbonates or citrates. If desired, saline solution can be used, with pH

adjustment and buffering. Optimal dosages may vary over a broad range from approximately 0.1 to 10 mg/kg of body weight depending upon the particular compound employed.

In general, the compounds of the present invention are believed useful in treating murine P388 and murine L1210 leukemias.

The following compounds were administered to mice innoculated by intraperitoneal injection with P-388 and L-1210 leukemia cells. A single dose of the test compounds was administered on the days indicated beginning on day 1, 24 hours after implantation of the leukemia cell. IP denotes intraperitoneal drug injection and IV denotes intravenous. Doxorubicin hydrochloride was administered for comparison.

Ten mice were employed in each test. The animals were observed and their survival compared with that of control animals which received the same leukemia innoculation but were not treated with drug. The results are shown in the Table where T/C is the ratio of animals surviving at 10 days which received drug divided by the number of surviving control animals.

TEST COMPOUNDS

| Compound No. | Identification |
|---|---|
| 1 | 14-0-acetyl-7-O-(3,4-di-0-acetyl-2,6-dideoxy-2-iodo-α-L-manno-hexopyranosyl adriamycinone |
| 2 | 14-O-Acetyl-7-O-(3,4-di-O-acetyl-2-bromo-6-dideoxy-α-L-talo-hexopyranosyl)adriamycinone |
| 3 | 14-O-Acetyl-7-O-(3,4-di-0-acetyl-2,6-dideoxy-2-iodo-α-L-talo-hexopyranosyl) adriamycinone |
| 4 | 14-O-acetyl-7-O-(3,4-di-0-acetyl-2-bromo-2,6-dideoxy-α-L-manno-hexopyranosyl)adriamycinone |
| 5 | 14-O-acetyl-7-O-(3,4-di-O-acetyl-2-chloro-2,6-dideoxy-α-L-manno-hexopyranosyl)-adriamycinone |

BAD ORIGINAL

0116222

TABLE B

| Tumor System | Test Compound No. | Route | Schedule | Dose (mg/kg) | T/C |
|---|---|---|---|---|---|
| P388 | 1 | IP | Day 1 | 50 | 54 |
| | | | | 37.5 | 171, 400 |
| | | | | 25 | 300, 171, 238 |
| | | | | 12.5 | 181, 152, 182 |
| | | | | 6.25 | 152, 134, 154 |
| | | | | 3.12 | 142, 115 |
| | | | | 1.56 | 111 |
| | Doxo-rubicin | | | 12.5 | 103 |
| | | | | 6.25 | 206, 249, 220 |
| P388 | 1 | IV | Day 1 | 20 | 156 |
| | | | | 15 | 141 |
| | | | | 10 | 136 |
| | | | | 5 | 120 |
| | Doxo-rubicin | | | 15 | 199 |
| L1210 | 1 | IP | Days 1,5,9 | 15 | 234 |
| | | | | 7.5 | 176 |
| | | | | 3.75 | 141 |
| | | | | 1.88 | 129 |
| | Doxo-rubicin | | | 3 | 158 |
| | | | | 2 | 148 |
| P388 | 2 | IP | Day 1 | 25 | 204 |
| | | | | 12.5 | 150 |
| | | | | 6.25 | 138 |
| | | | | 3.12 | 117 |
| | Doxo-rubicin | | | 6.25 | 209 |
| P388 | 3 | IP | Day 1 | 25 | 172 |
| | | | | 12.5 | 147 |
| | | | | 6.25 | 126 |
| | | | | 3.12 | 114 |
| | Doxo-rubicin | | | 6.25 | 209 |

| Tumor System | Test Compound No. | Route | Schedule | Dose (mg/kg) | T/C |
|---|---|---|---|---|---|
| P388 | 4 | IP | Day 1 | 50 | 318 |
| | | | | 25 | 238, 220 |
| | | | | 12.5 | 218 |
| | | | | 6.25 | 172 |
| | | | | 3.12 | 147 |
| | Doxo-rubicin | | | 6.25 | 209 |
| L1210 | 4 | IP | Days 1,5,9 | 15 | 234 |
| | | | | 7.5 | 171 |
| | | | | 3.75 | 141 |
| | | | | 1.88 | 127 |
| | Doxo-rubicin | | | 4 | 158 |
| P388 | 5 | IP | Day 1 | 25 | 254 |
| | | | | 12.5 | 249 |
| | | | | 6.25 | 195 |
| | | | | 3.12 | 173 |
| | Doxo-rubicin | | | 5 | 276 |
| L1210 | 6 | IP | Days 1,5,9 | 15 | 342 |
| | | | | 7.5 | 184 |
| | | | | 3.75 | 138 |
| | | | | 1.88 | 120 |
| | Doxo-rubicin | | | 4 | 158 |

Having described the invention in detail and by reference to specific embodiments thereof, it will be apparent that numerous variations are possible without departing from the spirit and scope of the invention defined by the following claims.

What is claimed is:

CLAIMS

1. A compound of the formula (I)

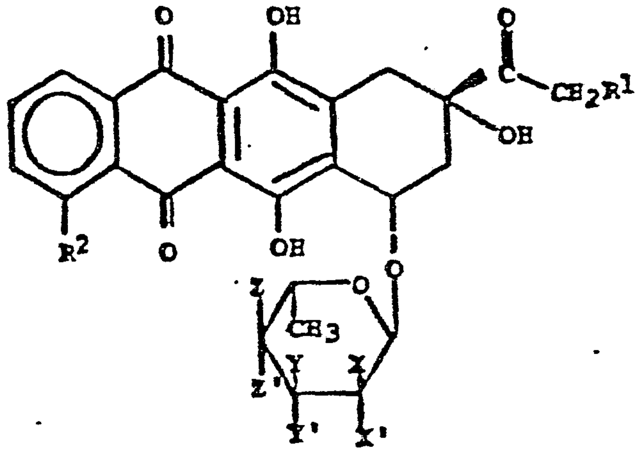

wherein $R^1$ is $-OOCR^3$ or $-OOC(CH_2)_nCOOR^4$; $R^2$ is hydrogen, hydroxy or methoxy; one of X and X' is a halogen atom selected from the group consisting of fluorine, chlorine, bromine and iodine and the other is hydrogen; one of Y and Y' is hydrogen and the other is selected from the group consisting of hydrogen, hydroxy and acyloxy; one of Z and Z' is hydrogen and the other is selected from the group consisting of hydrogen, hydroxy and acyloxy; $R^3$ is an alkyl group containing 1 to 8 carbon atoms; $R^4$ is a hydrogen atom, a metal atom or an alkyl group containing 1 to 4 carbon atoms and n is an integer of 0-6, and pharmaceutically acceptable salts thereof.

2. The compound of claim 1 wherein $R^1$ is $-OOCR^3$ wherein $R^3$ is an alkyl group containing 1 to 8 carbon atoms.

3. The compound of claim 1 wherein $R^1$ is $-OOC(CH_2)_nCOOR^4$ and $R^4$ is hydrogen or a lower alkyl group containing 1 to 4 carbon atoms or an alkali metal atom.

4. The compound of claim 2 wherein X'is bromine or iodine.

5. The compound of claim 4 wherein X' is bromine or iodine, Y' is acetoxy or hydroxy and Z is acetoxy or hydroxy.

6. The compound of claim 3 wherein X'is bromine or iodine.

7. The compound of claim 6 wherein X' is bromine or iodine, Y' is acetoxy or hydroxy and Z is acetoxy or hydroxy.

8. A pharmaceutical preparation comprising a therapeutically effective amount of a compound of the formula (I)

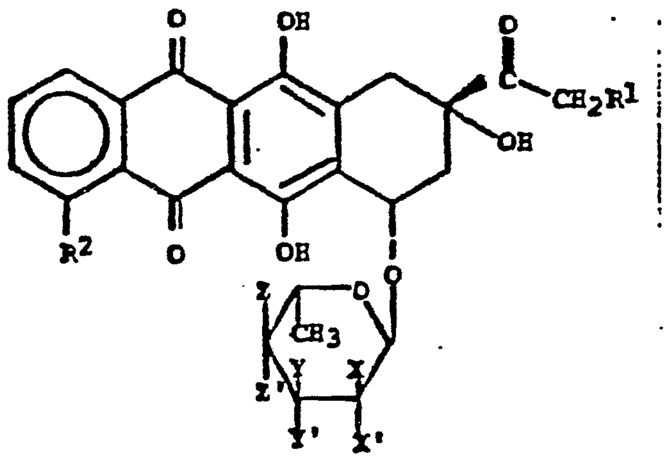

wherein $R^1$ is $-OOCR^3$ or $-OOC(CH_2)_nCOOR^4$; $R^2$ is hydrogen, hydroxy or methoxy; one of X and X' is a halogen atom selected from the group consisting of fluorine, chlorine, bromine and iodine and the other is hydrogen; and one of Y and Y' is hydrogen and the other is selected from the group consisting of hydrogen, hydroxy and acyloxy; one of Z and Z' is hydrogen and the other is selected from the group consisting of hydrogen, hydroxy and acyloxy; $R^3$ is an alkyl group containing 1 to 8 carbon atoms; $R^4$ is a hydrogen atom, an alkali metal atom or an alkyl group containing 1 to 4 carbon atoms and n is an integer of 0-6 in combination with a pharmaceutically acceptable carrier.

9. The pharmaceutical preparation of claim 8 wherein $R^1$ is $-OOCR^3$ wherein $R^3$ is an alkyl group containing 1 to 8 carbon atoms.

10. The pharmaceutical preparation of claim 8 wherein $R^1$ is $-OOC(CH_2)_nCOOR^4$ and $R^4$ is hydrogen or a lower alkyl group containing 1 to 4 carbon atoms or an alkali metal atom.

11. The pharmaceutical preparation of claim 9 wherein X' is bromine or iodine.

12. The pharmaceutical preparation of claim 11 wherein X' is bromine or iodine, Y' is acetoxy or hydroxy and Z is acetoxy or hydroxy.

13. The pharmaceutical preparation of claim 10 wherein X' is bromine or iodine.

14. The pharmaceutical preparation of claim 13 wherein X' is bromine or iodine, Y' is acetoxy or hydroxy and Z is acetoxy or hydroxy.

European Patent Office

# EUROPEAN SEARCH REPORT

0116222

Application number

EP 83 30 7715

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | WO-A-8 000 305 (UNITED STATES GOVERNMENT)<br>* Pages 26,27 *<br>----- | 1,8 | C 07 H 15/24<br>A 61 K 31/00 |
| | | | TECHNICAL FIELDS SEARCHED (Int. Cl. 3) |
| | | | C 07 H 15/00<br>A 61 K 31/00 |

The present search report has been drawn up for all claims

| Place of search | Date of completion of the search | Examiner |
|---|---|---|
| THE HAGUE | 04-04-1984 | VERHULST W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82